# EUROPEAN PATENT SPECIFICATION

(11) **EP 3 473 284 B1**
(45) Date of publication and mention of the grant of the patent: **01.10.2025**
(21) Application number: 18000270.1
(22) Date of filing: 19.03.2018
(51) Int. Cl.: A61M 5/28, A61M 5/24, A61M 5/31, A61J 1/20, A61M 3/00, A61M 5/315

(54) **ELASTIC AND SLIDING VALVULAR JOINT, SUITABLE TO WORK IN PRE-FILLED SYRINGES AND SAID SYRINGES**
ELASTISCHES UND GLEITENDES VENTILGELENK, GEEIGNET ZUM ARBEITEN IN VORGEFÜLLTEN SPRITZEN UND DIESE SPRITZEN
JOINT VALVULAIRE ET COULISSANT, ÉLASTIQUE POUVANT FONCTIONNER DANS DES SERINGUES PRÉ-REMPLIES ET LESDITES SERINGUES

(30) Priority: 19.10.2017 AR P010290217
(43) Date of publication of application: 24.04.2019
(73) Proprietor: Szapiro, Jaime Luis, Ciudad de Buenos Aires (AR); Moreno Bonino, Saúl, Provincia de Buenos Aires (AR); Szapiro, Germán Andrés, Ciudad de Buenos Aires (AR)
(72) Inventor: Szapiro, Jaime Luis, Ciudad de Buenos Aires (AR); Moreno Bonino, Saúl, Provincia de Buenos Aires (AR); Szapiro, Germán Andrés, Ciudad de Buenos Aires (AR)
(74) Representative: Fernández-Pacheco, Aurelio Fernández

(56) References cited:
- EP-A1- 0 974 373
- EP-A1- 1 213 036
- WO-A1-2018/134211
- AR-A3- 100 575
- CN-B- 102 861 369
- US-A- 5 643 224

## Description

### FIELD OF THE INVENTION

The present invention makes reference to the field of syringes to apply injections, preferably to those pre-filled and disposable syringes, and even more particularly to an elastic and sliding valvular joint for pre-filled disposable syringes to maintain the contents therein completely isolated and sterile, preventing them to reach the needle before the injection.

### BACKGROUND OF THE INVENTION

Generally, the pre-filled syringes include a cylindrical and hollow main body, with a front section where a communication neck is formed with the injection needle; while on the inside there is a plunger coaxially arranged and manually movable. This plunger extends outwards from the base opposite to the neck, which is totally open.

Under these conditions, an internal chamber of variable volume is defined. This chamber constitutes the temporary location for the product to be injected. Said chamber is determined by the cylindrical wall of the main body, the cited front neck, where the needle couples, and the active head of the plunger manually movable that is exactly supported by the internal face of said cylindrical wall of the main body.

The needle is housed in the interior of a protective sheath, which at the same time locks and couples to it keeping it isolated and steady, while it also couples to the syringe itself producing a temporary hermetic closure.

Taking into consideration the basic structure previously described, there are several embodiments where the mentioned interior of the main body is also subdivided in two chambers of variable volume that are adjacent and coaxial, and separated between them by internal transversal partitions, which include valvular means for the communication between both chambers.

It makes reference to the usually called pre-filled double-chamber syringes, which are used for those cases where two different liquid products are stored, or when one liquid product and another powdered product must be kept completely isolated and separated one from the other until the moment of injection, where they are previously mixed.

Indeed, in the case of double-chamber syringes, said internal valvular means, which constitute the partition that separates both chambers, are designed for the user to mix the products before the injection without being contaminated by the outer environment. That is to say, it maintains the isolation from origin. It is then that the protective sheath can be removed from the needle and, the resulting mix can be moved to the needle, initiating the injection process.

In order to carry out said mixing step, while maintaining the mentioned isolation, it is usual and common to use the plunger of the syringe itself moving it in a direction opposite to the direction of the injection. This moving action generates pressures and depressions in said adjacent chambers; and therefore, the mentioned internal valvular means open, establishing a communication that allows one of the products to pass and mix with the other, and then placing the mix in only one chamber and in the conditions to be injected.

Once the mix is made the same user shall only proceed with the injection for the mix to move forward towards the needle.

There are several constructive embodiments that define said internal valvular partitions, which separate the adjacent chambers that contain the separated products. Precisely, they differ by the valvular resource they include in each case.

However, from the analysis of these well-known internal valves, there arises the problem of the valvular means especially designed for each particular case. That is to say, they can only act in the body of a syringe that is structurally and functionally adapted to them.

In other cases, there are valvular elements that can be adapted to the body of conventional syringes, but which require the addition of an accessory or a structural modification to the body of the syringe, which raises their final price and complicates their manufacture and filling.

It is further clarified that in order for these valvular joints to act effectively in pre-filled double-chamber syringes, it is important that the mentioned neck of the syringe, where the injection needle couples with its protective sheath, remains closed by a valve-plug or, otherwise by an adequate element that keeps the liquid isolated in the interior of the needle until the injection. In this manner, it guarantees that the coupling and uncoupling actions shall not cause an unwanted loss or spill.

Within this type of valvular joints, it is possible to find the Argentine Patent of Invention N° AR 026723 B1(=EP 1213036 B1, ES 2254321 T3) who owners are Jaime Luis Szapiro, Saúl Moreno and Leonardo Szames and, it is entitled "ELASTIC AND SLIDING VALVULAR JOINT, SUITABLE TO WORK IN PRE-FILLED SYRINGES" ("CONJUNTO VALVULAR ELÁSTICO Y DESLIZANTE APTO PARA ACTUAR EN EL INTERIOR DE JERINGAS PRELLENADAS"). This invention belongs either to the internal single-chamber type, which stores the product to be injected, or to the internal and independent double-chamber type, which holds the corresponding isolated products that must be mixed before the injection. This valvular joint acts as a temporary closure that prevents said internal chambers from communicating between them or with the communication tube towards the injection needle.

The joint consists of two discoid cooperative elements arranged inside the main body of the syringe, where their perimeter edges are supported by the cylindrical surface of the body. One of said elements is an elastic and sliding discoid valve, while the other constitutes the sliding discoid seat on which said discoid valve acts. This discoid valve has an elastic base with multiple holes from where a stub is formed. This stub is opposite to a corresponding passage hole that is defined in the base of said sliding discoid seat.

The perimeter edges of the bases of both discoid cooperative elements determine thicker and less elastic cordons than the rest of the body of each valvular element.

According to this disclosure, the mentioned discoid cooperative elements, which are arranged above the internal communication between the main body and the neck of the syringe, may act as a resource of temporary closure for pre-filled single-chamber syringes; or if they are combined with a valvular plug that closes the neck of the syringe, they may act as a resource of temporary closure for pre-filled syringes with two internal coaxial chambers, acting as a separating partition between them.

It has been verified that in this type of disposable syringe, after mixing the product to be injected and moving the plunger of the syringe to perform the injection, the mentioned elastic and sliding discoid valve, which holds the closing stub of the passage that defines the sliding discoid seat, releases said passage. However, it tends to return to its closing position.

In effect, in some cases, when the user proceeds with the injection, he/she detects that the mentioned closing stub wrongfully enters the passage hole of the discoid seat, acting as an unexpected plug and generating the need of repeating the initial handling by moving the plunger backwards again and, hence releasing the passage again.

This happens due to the mentioned closing stub that keeps aligned with the referred passage hole. Although it has a diameter slightly bigger than said hole, it generates an undesired blockage when it settles due to the pressure produced by the plunger.

In this case it is sufficient to restart the filling action by moving the plunger of the syringe in an opposite direction to the direction of the injection in order for the stub to uncouple.

Likewise, given the design of the valvular joint described in this document, only thermoplastic injection elements can be manufactured. This means that only an elastomeric product that allows injection moulding, such as santoprene, can be used as a building material. This type of product, once injected, has a relatively short useful life as it tends to lose its elastic memory over time. This phenomenon generates the possibility that the elements that compose the invention do not separate properly, or that once separated they often tend to reassemble easily. This is to the detriment of the functioning of the invention.

Likewise, patent application EP0974373A1 (= ES2211014T3) refers to a syringe of the type that has two variable volume chambers inside the main cylindrical body, these chambers are separated by a displacement valve assembly and are capable of housing components that must be mixed before injection. The novelty lies in the special conformation of parts and elements that make up this valve assembly, which comprises two portions of elastomer that cooperate and displace, one of these parts being comprised by a hollow peripheral cylindrical body that rests on the cylindrical wall of the main body, the other part being a solid, internal cylindrical body that slides on the inside face of the front.

The internal part of the valvular joint described in this document is solid and has such a conformation that it is inserted into the body of the first part of the valve, which rests on the inner cylindrical wall of the syringe. Its arrangement and conformation is intended to avoid displacements and to keep the whole aligned during the embolade since it is a solid body of little flexibility and must allow the flow of liquid freely during the mixing of components and then its exit towards the needle.

The complexity of the elements of the valvular joint described in this document prevents the versatility of materials and processes that facilitate their proper manufacture and optimal operation.

Moreover, patent US5643224A (= ES2087835B1) refers to a stopper for making pre-filled syringes, which is a distinct and completely different device that has been conceived for another purpose. In short, it has nothing to do with the primary and secondary inventive object being processed in this application.

In effect, this document refers to a safety valve plug, applicable to pre-filled disposable syringes, which consists of a solid, substantially cylindrical body with a conical end and, in the other section that defines its head, a cylindrical area with a larger cross-section than the rest of the body, with the conical area following, two facetted faces extending to the proximal area of the head, the facetted faces being equal to each other, parallel and symmetrical as well as coaxial to the longitudinal axis of the stopper, and the cylindrical head having two facetted faces equal and parallel, symmetrically arranged, the facetted faces of the cylindrical body being parallel to the faces of the cylindrical head. Therefore, this patent refers to a plug that functions exclusively in the outlet of any syringe and does not constitute a valvular joint for internal use in pre-filled syringes.

In Patent WO 2018/134211, the central tube of second engaging means is in fact provided of the piston of the syringe. However, in the present application, the valvular joint is an entity separate from and freely sliding with respect to the piston of the piston of the prefilled syringe.

Within this type of valvular joints we find also the Argentine Patent of Invention AR 082099 A1 (=CN 102861369 A), owned by Jaime Luis Szapiro, Saúl Moreno and Leonardo Szames, and entitled "ELASTIC AND SLIDING VALVULAR JOINT FOR DISPOSAL PRE-FILLED SYRINGES" ("CONJUNTO VALVULAR ELÁSTICO Y DESLIZANTE PARA JERINGAS PRELLENADAS DESCARTABLES"), makes reference to a valvular joint suitable to be used either in pre-filled syringes of a single internal chamber type that stores the product to be injected, or in those of two independent internal chambers that contain the corresponding isolated products to be mixed before the injection. This valvular joint acts as a temporary closure that prevents said internal chambers from communicating between them, or with the communication tube towards the injection needle. Said valvular joint consists of two cooperative discoid elements arranged inside the main body of the syringe, with their perimeter edges supported by its cylindrical surface. One of said elements is a sliding and elastic discoid plug, while the other consists of a sliding receiving discoid seat, on which said plug acts. The perimeter edges of the bases from both discoid elements determine each cordon, which are thicker and less elastic than the rest of the body of each valvular element.

The sliding and elastic discoid plug consists of an elastic base with multiple holes; and from its internal face a hollow closing cylinder with an open distant base is formed. Moreover, the sliding receiving discoid seat includes a central passage tube opposite to said closing cylinder, which has superficial cavities in its internal opening that maintain the communication with the internal tube.

Additionally, the closing cylinder formed from the elastic base of the valvular plug has a circular section, which has an external diameter that is slightly bigger than the diameter of the passage tube of the receiving discoid seat.

Due to the design of the valvular joint described in this document, its elements can only be produced by thermoplastic injection. For this reason, only an elastomeric product that allows injection moulding, such as santoprene, can be used as a building material. Once injected, the elements of this valve assembly have a relatively short service life as they tend to lose their elastic memory in time. This phenomenon generates that these elements that compose the valve assembly are not separated adequately, or that once separated often tend to reassemble easily. This fact is detrimental to the functioning of the invention.

Patent application AR 100575 A3 concerns a valvular resource of the type comprising two disc elements of co-operative action, which are placed inside the main body of the syringe with its perimeter edges resting on the cylindrical surface of the syringe. One of them is an elastic and sliding disc plug, while the other is the receiver disc seat on which the plug acts, where the perimeter edges of the bases of both disc elements determine cords that are thicker and less elastic than the rest of the body of each valve element. The elastic sliding disc plug comprises a closed flat base which, from its internal face, projects a hollow sealing cylinder whose distal base is open, while its thicker perimetral cord is affected by peripheral lateral grooves constituting the respective product passage ducts during its operation. On the thickest perimetral cord, two or more peripheral lateral grooves located equidistant from each other are defined, which are of the same height as the thickness of the same.

This application describes a valve resource in an attempt to achieve a valve assembly independent of a single material, such as santoprene, and of an exclusive method of manufacture. It is also intended to eliminate the perforations in the flat body of the disc comprising the plug by two perimeter grooves. This resource weakens the perimeter edge of the disc which must be thicker and less elastic than the rest of the body and therefore affects its functioning. Likewise, the passage of product through the aforementioned grooves is not substantially improved. Specifically, versatility of materials and method of manufacture was sought and the flow rate improved through the perimeter grooves, but the perimeter adjustment cord of the disk against the syringe wall was weakened.

Therefore, it is necessary to have a valvular joint with a simplified design that allows a more effective functioning and, instead of needing the manufacturing of the elements of the valvular joint only by injection of an elastomer, such as Santropene, (which is a thermoplastic rubber to be processed on plastics machinery but with the final appearance and features of rubber), it allows the use of different methods of manufacturing, such as the elastomer compression molding and other types of cheaper materials for medical use. This may be the case of butyl rubber, which is a synthetic copolymer rubber of isobutylene with isoprene ("IIR"- Isobutylene Isoprene Rubber, for its acronym in English), widely used in current pharmacotechnics.

Moreover, when the solution to be injected (for example, powder with a dissolved active in a suitable solvent) and resulting from the mixing process moves towards the upper chamber of a pre-filled syringe, or when the solution to be injected from a pre-filled syringe is in a single chamber, it is necessary to have a valvular joint that allows the free flow of the solution to be injected through a wide tube and not through small holes; so that consequently and with the upthrust of the plunger, the components of the valvular joint can act together and assemble with the plunger in the first case, or they can assemble again when finalizing the injection in the second case.

### SUMMARY OF THE INVENTION

The invention is defined in claim 1. Advantageous embodiments are subject of the dependent claims.

Therefore, the main object of this invention is a pre-filled syringewith a single-chamber type that stores the product to be injected, or in those of two independent internal chambers that contain the corresponding isolated products to be mixed before the injection, and which contains a sliding and elastic valvular joint suitable to work in the interior of the cited pre-filled syringe wherein this valvular joint acts as a temporary closure that prevents an internal chamber from communicating with the communication tube towards the injection needle or between both internal chambers, containing-said valvular joint two cooperative discoid elements arranged inside the main body of the syringe, with its perimeter edge supported by its cylindrical surface, wherein one of said elements is an elastic discoid plug, while the other consists of a sliding receiving discoid seat, on which said plug acts through a cylindrical central passage tube wherein the perimeter edge of the discoid seat consists of a cordon, which is thicker and less elastic than the rest of the body of each discoid seat wherein the elastic discoid plug of said valvular joint consists of an elastic base and, from its internal face a closing hollow cylinder is formed with an open distant base while the sliding receiving discoid seat includes a central passage tube facing the closing hollow cylinder, in such a way that the elastic base of the discoid plug fits in a removable manner inside a cylindrical cavity of the opening of the central passage tube of the sliding receiving discoid seat wherein the diameter of the elastic base of the elastic discoid plug is equivalent to the diameter of the sliding receiving discoid seat and when the hollow closing cylinder is inside the central passage tube of the sliding receiving discoid seat, said elastic base of the elastic discoid plug fits inside a circular cavity surrounded by the perimeter edge of the discoid seat that forms the thicker cordon, and where the external diameter of the hollow closing cylinder is equivalent to the internal diameter of the central passage tube of the sliding receiving discoid seat offering a calibrated fit, wherein the discoid cooperative elements of the valvular joint are manufactured in an elastomeric material indistinctly by injection or by compression.

The closing hollow cylinder that is formed from the elastic base of the elastic discoid plug of the elastic valvular joint has a circular section, where the external diameter is slightly bigger than the diameter of the central passage tube of the sliding receiving discoid seat, offering a calibrated fit.

In another alternative way, the closing hollow cylinder that is formed from the elastic base of the valvular plug of the elastic valvular joint includes a plurality of external annular flanges that are temporarily supported by the cylindrical surface defined by the central passage tube from the sliding receiving discoid seat, offering a calibrated fit.

Additionally, the central passage tube of the sliding receiving discoid seat of the elastic valvular joint includes in its opening at least two superficial adjacent cavities facing each other, which communicate with the interior of the central passage tube of the sliding receiving discoid seat.

Preferably, the cooperative discoid elements of the elastic valvular joint are combined with an upper closure plug (tip-cap) that closes the communication tube towards the injection needle, which is not pre-installed in its position, act as a temporary closure for pre-filled syringes of two internal coaxial chambers, where said cooperative discoid elements are arranged as a separating partition between said chambers.

It is also preferably that the cooperative discoid elements of the elastic valvular joint that are combined with a valvular plug that closes the communication tube towards the injection needle, which is pre-installed in its position, be a temporary closure for pre-filled syringes of two internal coaxial chambers, where said cooperative discoid elements are arranged as a separating partition between said chambers.

In a preferred embodiment, the cooperative discoid elements of the elastic valvular joint, which are arranged as a temporary closure that prevents the internal communication of the main body of the syringe with the communication tube towards the injection needle, act as a temporary closure for pre-filled syringes of a single chamber.

In another embodiment, the present invention refers to a pre-filled syringe that consists of an elastic valvular joint according to any of the above claims, forming two chambers, where each one of them holds a different product being at least one of them liquid; and where both products after being mixed result in a solution to be injected, and the elastic discoid plug of the elastic valvular joint is opposite to the head of the plunger.

Particularly, when the injection needle is pre-installed in the exit tube of the syringe, said tube is closed by an internal valvular closure plug.

Particularly the elastic valvular joint, forms a chamber that includes a solution to be injected, where the elastic discoid plug of the elastic valvular joint is opposite to the head of the plunger.

Particularly, the injection needle is pre-installed in the exit tube of the syringe.

### BRIEF DESCRIPTION OF THE DRAWINGS

Despite the advantages the users or specialists could add to this invention, aiming to specify the advantages cursory mentioned above, and in order to facilitate the understanding of the structural and functional characteristics of this invented elastic valvular joint, a preferred embodiment is described below and illustrated in a schematic manner and without a specific scale, in the sheets of drawings attached herewith. It is important to state clear that since it is a preferred embodiment, its scope of protection should not be considered limiting or exclusive. Instead, it is simply aimed to explain and illustrate the basic understanding upon which the invention is based.
Figure 1. is a cross-section view that shows the separated elements that form the elastic valvular joint of this invention.
Figure 2. is a cross section view, which is similar to the previous figure and, in this case shows the same elements coupled between them.
Figure 3. is a perspective view that represents the elastic discoid valvular plug that constitutes one of the two elements from the invented valvular joint as seen from its external face.
Figure 4. is a perspective view that represents the elastic discoid valvular plug that constitutes one of the two elements from the invented valvular joint as seen from its internal face.
Figure 5. is a perspective view that represents the sliding receiving discoid seat that constitutes the other element from the invented valvular joint as seen from its external face.
Figure 6. is a perspective view that represents the sliding receiving discoid seat that constitutes other element from the invented valvular joint as seen from its internal face.
Figure 7. is a perspective view that represents the other preferred embodiment of a sliding receiving discoid seat that constitutes the other element from the invented valvular joint, as seen from its internal face, where two opposite tubes can be seen in the opening of the cylindrical tube.
Figure 8. is a vertical lengthwise cross-section view that represents a pre-filled double-chamber syringe of conventional variable volume, in which interior the valvular joint of this invention is arranged at a rest position as it is offered in the commerce.
Figure 9. is also a lengthwise cross-section view, which is similar to the previous figure, and in this case it shows the invented valvular joint in the position after the mixing process is initiated inside the syringe.
Figure 10. is also a lengthwise cross-section view, which is similar to the previous figures, and in this case it shows the invented valvular joint in the position after the mixing process is finished inside the syringe.
Figure 11. 12 and 13 are lengthwise cross-section views, which are similar to the previous figures, and in this case they gradually show the arrangement of the two valvular elements that belong to the invented joint when the resulting mix is moved to be placed in conditions suitable to be injected.
Figures 14 and 15 are lengthwise cross-section views, which are similar to the previous figures, and in this case they show the arrangement of the valvular elements that belong to the invented joint when injection is occurring.
Figure 16 is a lengthwise cross-section view that shows the simple-chamber syringe of conventional variable volume, where the invented valvular joint is also included. However, in this case, the joint acts as a seal that prevents the content of the syringe from entering the needle channel.
Figure 17 is a lengthwise cross-section view similar to the previous Figure 16, which shows the performance of the valvular elements when applying F force with the plunger outside the pre-filled syringe and moving the plug of the valvular joint before the injection process.
Figure 18 is a lengthwise cross-section view similar to the previous Figure 16, which shows the performance of the valvular elements when the product of the pre-filled syringe is moved through the syringe and the needle during the injection process.
Figure 19 is a vertical lengthwise cross-section view that represents a double-chamber pre-filled syringe of conventional variable volume, in which interior the valvular joint of this invention is arranged at a rest position as provided in the commerce, with the injection needle previously installed in its protective sheath and the valvular closure plug that prevents the product to be injected and inside the syringe from communicating with the injection needle.
Figure 20 is a vertical lengthwise cross-section view that represents a double-chamber pre-filled syringe of conventional variable volume from Figure 18, in which interior the valvular joint of this invention acts allowing the mix of the products arranged in separate chambers, and there being an injection needle previously installed in its protective sheath, and a valvular closure plug preventing the product to be injected and inside the syringe from communicating with the injection needle.
Figure 21 is a vertical lengthwise cross-section view that represents a double-chamber pre-filled syringe of conventional variable volume from Figure 18, showing the performance of the elements of the valvular joint of this invention when the product of the pre-filled syringe is moved during the injection process towards the pre-installed injection needle without its protective sheath, and where the valvular-closing plug allows the communication of the product inside the syringe with the injection needle.

It is further clarified that in all the figures the same reference numbers correspond to the same or equivalent parts or elements forming the joint, according to the embodiment selected for this explanation of the invented valvular joint.

### DETAILED DESCRIPTION OF THE INVENTION

The pre-filled syringes of this invention contains a valvular joint which belongs to the group of those adaptable to any type of body of the conventional syringe, whether single-chamber or double-chamber.

The constructive and functional design of this valvular joint takes into account the condition of adapting to any type of conventional syringe, the non-alteration of automatized processes for getting the products inside the syringe, especially for the cases of double-chamber pre-filled syringes, and also the lack of special and/or complicated tasks demanded to the user when mixing and later injecting.

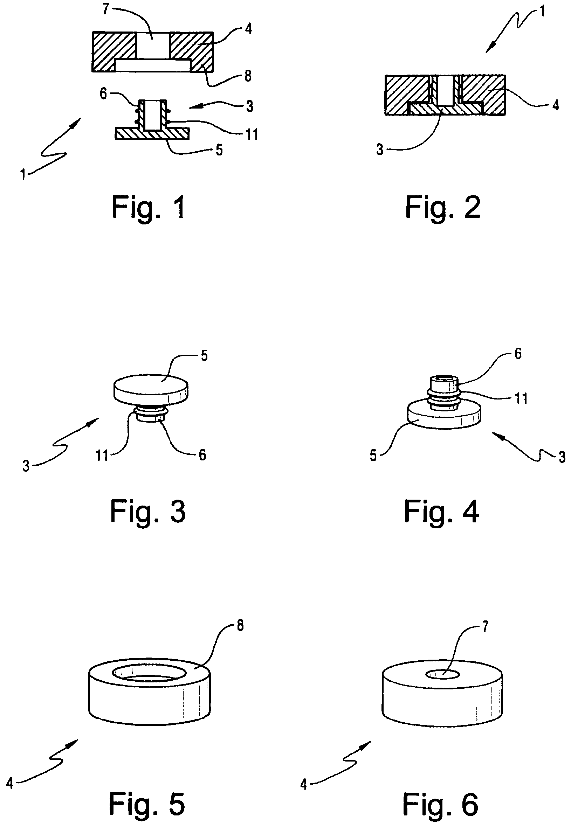

As it is noticed in Figures 1 to 7, the elastic and sliding valvular joint (1) for syringes (2) previously filled and referred to in this invention consists of two mutually cooperative elements represented by (3 and 4).

The one indicated with reference (3) is an elastic discoid plug that consists of an elastic discoid base (5) substantially formed in a cylindrical manner.

This elastic discoid plug (3) stands out especially since a closing hollow cylinder (6), which is coaxial with the mentioned elastic discoid base, is formed from its internal face and, its distant face is totally open.

The second element, a receiving discoid seat (4), has been created to allow the elastic discoid plug (3) to produce the closure or opening of the valvular joint (1), acting as a cooperative receiving discoid seat. This element is also preferably circular and it includes a central passage tube (7) and its corresponding perimeter ring (8) that is thicker than the rest of the body of said element.

As an alternative embodiment to the invention, this sliding receiving seat (4) is characterized especially due to the opening of its central passage tube (7), which is opposite to the closing hollow cylinder (6) of the elastic discoid plug (3) from the valvular joint (1). Furthermore, it preferably includes at least two superficial adjacent cavities (9 and 10) of short length, and each one of them constitutes a channel that communicates with the mentioned central passage tube (7).

The elastic discoid plug (3) and the receiving discoid seat (4) of the valvular joint (1) are cooperative elements because they must necessarily act together for the valvular opening and closing actions.

To this extent, said elements (3 and 4) must be overlapped and aligned between them inside the syringe; so that the mentioned closing cylinder (6) perfectly fits the interior of the central passage tube (7) generating the hermetic closure that prevents the products contained inside the syringe (2) previously filled from moving through said tube.

Said proper fit in the interior of the central passage tube (7) is obtained through a bigger diameter of the closing hollow cylinder (6).

As an alternative embodiment, said bigger diameter of the closing hollow cylinder (6) can be obtained through a plurality of external annular flanges (11), which are temporary supported by the cylindrical surface that defines the central passage tube (7) of the sliding receiving discoid seat (4).

As an alternative to this invention, in case the closing hollow cylinder (6) of the elastic discoid plug (3) is arranged to be supported by the central passage tube (7) of the receiving discoid seat (4), at least two of the mentioned superficial cavities (9 and 10) guarantee the flow of the fluid towards the exit of the syringe (2) during the injection without drawbacks.

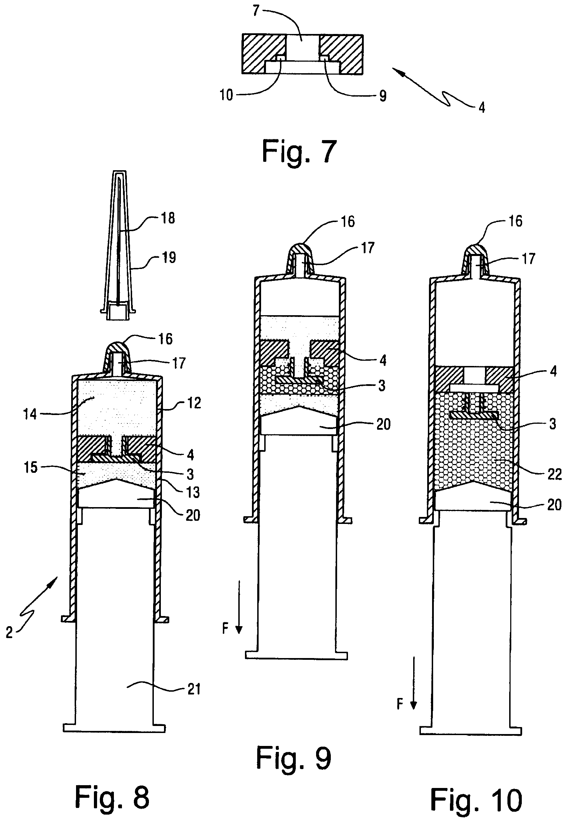

As from Figures 8 to 13, it is possible to understand how the invented joint acts when it is applied to a syringe (2) previously filled with two chambers (12 and 13) during the process of mixing of products (14 and 15) contained in both chambers (12 and 13).

Indeed, in Figure 8 it is possible to observe, in a lengthwise cross-section view, a syringe (2), which is conventional and pre-filled as it is before the injection conditions, i.e. with the corresponding products (14 and 15) duly separated and isolated between them and with the exterior and stored in the respective chambers (12 and 13) of variable volume inside the body of the syringe (2).

By means of an upper closure plug (16), also known as "tip-cap", which is in the exit tube (17) of the syringe (2), it is possible to gurantee the airtightness and hence, the duly isolation of the commercial product.

As it is already known, the syringe (2) includes its corresponding needle (18) of injection, which is covered by a protective sheath (19), which usually accompanies the commercial product in its container.

In said Figure 8 it can be clearly identified that the upper chamber (12) of the syringe (2), which is closer to the exit of the product during the injection, is delimited in its base by the valvular joint (1) of the invention, in its sides by the internal face of the cylindrical wall of the main body of the syringe (2) and in the upper part by the mentioned closure plug (16), containing the first product (14) duly isolated.

Furthermore, the lower chamber (13) is delimited in its upper part by the same valvular joint (1), by the internal face of the cylindrical wall of the main body of the syringe (2) and by the head (20) of the plunger (21), containing the second product (15) duly isolated.

In the conditions mentioned above, as it is shown in Figure 8, it is possible to store in said chambers (12 and 13) two products, the first one (14) and the second one (15), which may be liquid or solid in powder-form; preferably, for example one liquid and one solid in powder-form, or two liquids. In both cases they are separately contained in any of the two chambers (12 and 13). Once the joint is initially formed, while observing said Figures 9 to 13 it is possible to notice the performance of the valvular joint (1) in this invention when the internal mixing action of the products (14 and 15) begins before the injection.

For this purpose the user moves the head (20) of the plunger (21) in the direction (F) opposite to the direction of the injection, generating a depression in the lower chamber (13), which consequently produces the movement of the elastic discoid plug (3) liberating the central passage tube (7) of the receiving discoid seat (4), and hence liberating the communication between both upper and lower chambers (12 and 13). This is possible because when said movement occurs, the product (14) flows through the central tube (7) of the receiving discoid seat (4).

In order to facilitate the obtaining of the solution (22) of the injection, it is preferably that the user vigorously shakes the syringe (2).

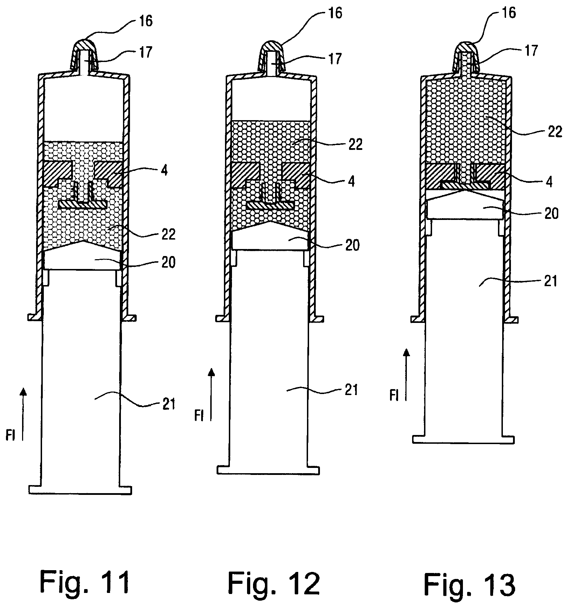

Figure 13 shows the arrangement of the referred elements (3 and 4) and the obtaining of the solution (22) to be injected by mixing the referred products (14 and 15), which is completely located in the upper distant chamber (12) and which is always duly isolated from the exterior by a closure plug (16).

It is emphasized the fact that the elastic discoid plug (3) is kept out of its position with respect to the central passage tube (7) of the receiving discoid seat (4), and the communication is kept between both internal chambers (12 and 13) during the actions to obtain the solution (22) to be injected.

Figures 11, 12 and 13 show that as a consequence of the communication set through the central passage tube (7), when the user moves the head (20) of the plunger (21) in the direction (FI) of the injection, the mentioned solution (22) moves from the lower or closer chamber (13) towards the upper or distant chamber (13) in a way that the solution is possible to be injected (22). Said solution was obtained in the same was as any other prefilled syringe (2) of a single conventional chamber.

The pressure made from the plunger (21) makes the closing hollow cylinder (6) of the elastic discoid plug (3) to settle on the opening of the central passage tube (7) and to fit it, which is why the valvular joint (1) works together during the injection of the content of the syringe (2), and in which case it does not affect the flow of fluid. This occurs because the fluid flowed perfectly and completely since said elastic discoid plug (3) did not completely close the tube (7).

In this respect, the closing hollow cylinder (6) has the same diameter or a bigger diameter than the central tube (7), which prevents the communication between the chambers (12 and 13) from closing until the direct pressure from the plunger (21) obliges the plug to returns to its initial position.

Notwithstanding the above, and as an alternative embodiment, the valvular joint (1) of this invention includes superficial cavities (9 and 10) adjacent to the opening of communication of the cited passage tube (7). So, in case said closing hollow cylinder (6) is supported by the opening of the passage tube (7), the solution fluid (22) normally flows through the mentioned cavities (9 and 10).

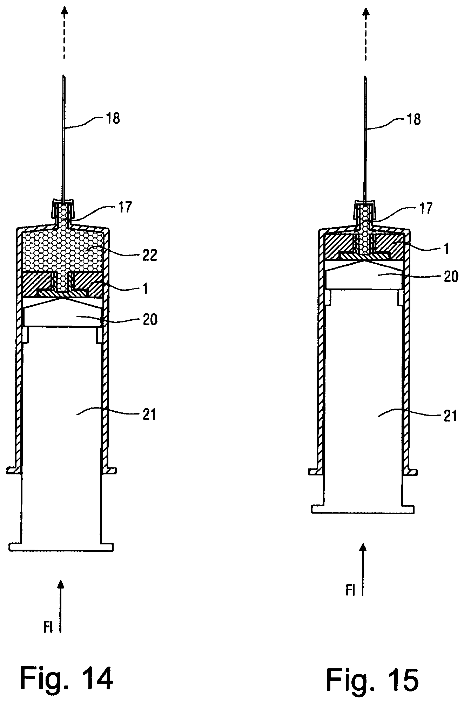

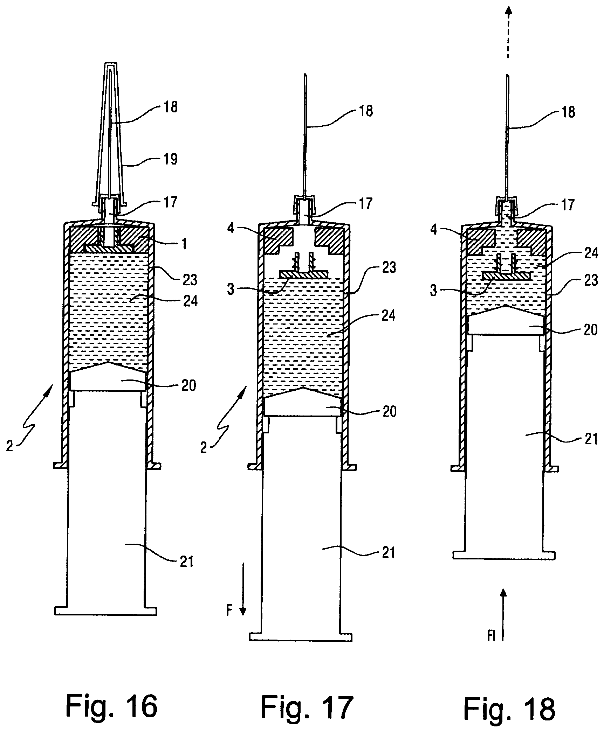

Figures 14 and 15 show that once the upper closure plug (16) is withdrawn and the injection needle (18) is placed in its position in the distant end of the exit tube (17) of the syringe (2), it will be sufficient for the user to continue moving the plunger (21) in the direction (FI) such that the pressure applied to the solution itself (22) makes it flow through the needle (19) of the injection by crossing it, after having previously removed the protective sheath (19) from said needle (18). Figures 16, 17 and 18 show the valvular joint (1) according to this invention included in a pre-filled conventional syringe (2) of only one chamber (23).

Indeed, for these cases the valvular joint (1) formed by elements (3 and 4) is placed in a way that it closes the internal communication of the syringe (2) with the exit tube (17) of the syringe (2), in such a manner that only the chamber (23) is delimited by the valvular joint (1) as the upper base, and by the cylindrical wall of the main body of the syringe (2) and the head (20) of the plunger (21) as the lower base.

In this case, the user also moves the plunger (21) in the inverse direction (F) of the injection producing an internal depression that generates the elastic deformation of the elastic discoid plug (3), and hence the movement of the closing hollow cylinder (6), which opens a communication through the central passage tube (7). Moreover, when applying an inverse force (FI), the injectable stored solution (24) can exit towards the needle (18) of the injection, and cross it after having previously removed the protective sheath (19) from said needle (18).

In both cases mentioned above, whether the pre-filled syringe (2) of two chambers (12 and 13) or of a single chamber (23), the internal communication of the syringe (2) through the exit tube (17) is initially and necessarily blocked by an upper closure plug (16) or "tip-cap", which is located in the exit tube (17) of the syringe (2), while the needle (18) of the injection is inside the protective sheath (19) and separated from the syringe (2) inside its corresponding packaging.

In the market there are syringes (2) previously filled, which are commercialized with the needle (18) being previously installed in their exit tube (17). Said syringes (2) may have two chambers (12 and 13) or one chamber (23) to contain two separate products that are mixed to obtain either one solution (22) to be injected or one injectable solution (24), respectively.

In the first case, the valvular joint (1) according to this invention may be used to separate both internal chambers (12 and 13), while the distant chamber (12) of the exit tube (17) of the syringe (2) of the needle (18) is separated by using the valvular closure plug (25) as it is described in the invention patent from Argentina 250777 V1, owned by Jaime Luis Szapiro, Leonardo Szames and Saúl Moreno, and entitled "VALVULAR SAFETY PLUG APPLICABLE TO DISPOSABLE PRE-FILLED SYRINGES" ("TAPON VÁLVULA DE SEGURIDAD APLICABLE A JERINGAS PRELLENADAS DESCARTABLES")

In this way, in those cases, where it is required to have a needle (18) of injection on top of the distant exit tube (17) of the syringe (2) previously filled, and when the valvular joint (1) of this invention must separate two chambers (12 and 13) with different products (14 and 15) to be mixed with the aim to obtain an injectable solution (22), said exit tube (17) must include a block, which may occur by means of a valvular closure plug (25) acting in the interior of the exit tube (17) of the syringe (2), where the injection needle (18) is already installed. Due to its special formation, said valvular closure plug (25) allows the exit of the solution (22) to be injected only when a force (FI) is applied producing an hydraulic pressure from the plunger (21) towards the syringe (2), which partially moves the valvular closure plug (25) and allows the flow of the solution (22) to be injected.

However, it is important to highlight that according to this invention, the aim and function of this closing valvular plug (25) does not affect the aim and function of the valvular joint (1). Instead, it is an alternative to the making of syringes (2) previously filled and of two chambers (12 and 13) that are separated by the valvular joint (1) according to this invention.

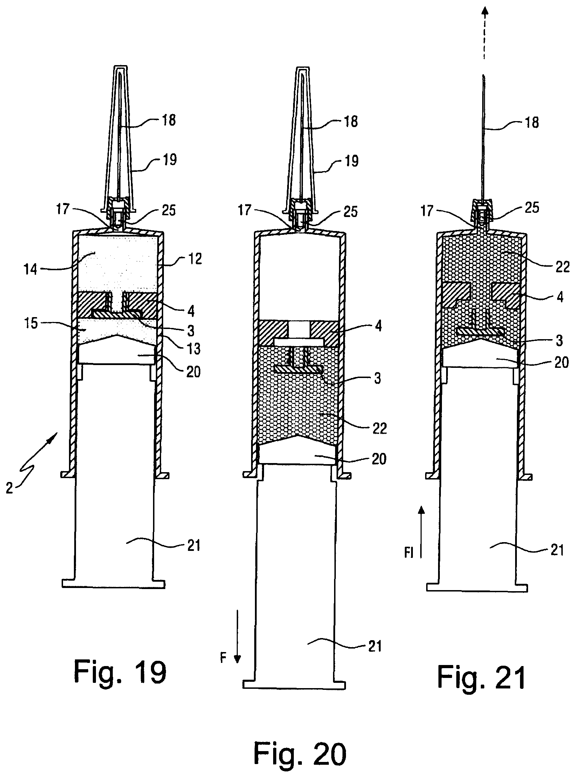

In Figure 19 it is possible to see a syringe (2) previously filled and of two chambers (12 and 13) with its corresponding needle (18) of injection previously installed and covered by its protective sheath (19), which is duly coupled to the exit tube (17) of the syringe (2) previously defined.

In Figure 20 it is possible to see an internal communication between the chambers (12 and 13), where said internal upper closing valvular plug (25) also acts guaranteeing the hermetic closure and hence, the duly isolation of the solution (22) to be injected.

As it can be seen in Figure 21, the closing valvular plug (25) is particularly characterized by the fact that it partially moves outwards allowing the exit of the solution (22) to be injected, and which was obtained from the mixing of products (14 and 15), through the needle (18). This movement occurs when internal pressure is generated during the action of injection by applying force (FI) on the plunger (21).

## Claims

1. A pre-filled syringe (2) that have one internal chamber (23) that stores the product to be injected, or two internal independent chambers (12 and 13) that contain isolated products (14 and 15), respectively, and which have to be mixed before the injection, comprising an elastic and sliding valvular joint (1) suitable to work inside said pre-filled syringe (2), wherein this valvular joint (1) is an entity separate from and freely sliding with respect to the piston of the pre-filled syringe, wherein this valvular joint (1) acts as a temporary closure blocking the communication between the internal chamber (23) and a communication channel (17) towards an injection needle (18), or between two internal chambers (12 and 13), said valvular joint (1) comprising two discoid cooperative elements arranged inside the main body of the syringe (2) and with its perimeter edge supported by its cylindrical surface, wherein one of said elements is an elastic discoid plug (3), while the other forms a sliding discoid receiving seat (4) on which said plug (3) acts through a cylindrical cavity (7), wherein the perimeter edge of the discoid seat (4) forms a cordon that is thicker and less elastic than the rest of the body of said discoid seat (4), said pre-filled syringe (2) **characterized in that** the elastic discoid plug (3) of said valvular joint (1) comprises an elastic base (5) and a hollow closing cylinder (6) with an open distal base, which is formed from its internal face, the sliding receiving discoid seat (4) includes a central passage tube (7) that faces said hollow closing cylinder (6) in a way that the elastic base (5) of the discoid plug (3) fits in a removable manner inside a cylindrical cavity located in the opening of the central passage tube (7) of the sliding receiving discoid seat (4), wherein the elastic base (5) of the elastic discoid plug (3) has a smaller diameter than the one from the sliding receiving discoid seat (4), and when the hollow closing cylinder (6) is inside the central passage tube (7) of the sliding receiving discoid seat (4), said elastic base (5) of the elastic discoid plug (3) is fitted inside a circular cavity surrounded by the perimeter edge (8) of the discoid seat (4) that forms the ticker cordon, and wherein the outer diameter of the hollow closing cylinder (6) is equivalent to the internal diameter of the central passage tube (7) of the sliding receiving discoid seat (4), offering a calibrated fit, wherein the discoid cooperative elements (3 and 4) of the valvular joint (1) are manufactured in an elastomeric material by injection or by compression, achieving that the versatility of having a valvular joint (1) for pre-filled syringes (2) with a single component to be injected or with two components to be mixed before injection, which does not need to be manufactured with a special material and by means of a single manufacturing process, which is elastic enough and with memory to allow that after separating the elements of the set of valves the contents of the syringe can be injected without inconvenience.

2. A pre-filled syringe (2) according to claim 1, **characterized in that** a hollow closing cylinder (6) formed from the elastic base (5) of the elastic discoid plug (3) can alternatively have a circular section, where the external diameter is slightly bigger than the diameter of the central passage tube (7) of the sliding receiving discoid seat (4), offering an adjusted fit.

3. A pre-filled syringe (2) according to claim 1, **characterized in that** the hollow closing cylinder (6) formed from the elastic base (5) of the valvular plug (3) can alternatively include a plurality of external annular flanges (11) that are temporary supported by cylindrical surface that defines the central passage (7) of the sliding receiving discoid seat (4), offering an adjusted fit.

4. A pre-filled syringe (2) according to any of the above claims from 1 to 3, **characterized in that** the central passage tube (7) of the sliding receiving discoid seat (4) comprises at least two superficial adjacent cavities (9 and 10) in its opening, which are opposed to each other and, which communicate with the interior of the central passage tube (7) of the sliding receiving discoid seat (4).

5. A pre-filled syringe (2) according to any of the above claims from 1 to 4, **characterized in that** the discoid cooperative elements are combined with an upper closure plug (tip-cap) (16) closing the communication channel towards the injection needle (18), which is not previous ly installed in its position, are a temporary closure resource for pre-filled syringes (2) of double internal coaxial chambers (12 and 13), where said discoid cooperative elements are arranged acting as a separating partition between said chambers (12 and 13).

6. A pre-filled syringe (2) according to any of the above claims from 1 to 4, **characterized in that** the discoid cooperative elements are combined with a valvular closure plug (25) internally closing the communication channel (17) towards the injection needle (18), which is pre-installed in its position, are a temporary closure resource for pre-filled syringes (2) of double internal coaxial chambers (12 and 13), where said discoid cooperative elements are arranged, acting as a separating partition between said chambers (12 and 13).

7. A pre-filled syringe (2) according to any of the above claims from 1 to 4, **characterized in that** the discoid cooperative elements that are arranged as a temporary closure that prevents the main body of the syringe (2) from communicating internally with the communication channel towards the injection needle (18), are a temporary closure resource for pre-filled (2) one chamber syringes.

8. A pre-filled syringe (2) according to any of the above claims from 1 to 7, **characterized in that** the elastic valvular joint (1) forms two chambers (12 and 13) that include one different product (14 and 15) each and, at least one of these products is liquid, wherein upon mixing the products (14 and 15), a solution (22) is obtained to be injected, wherein the elastic discoid plug (3) of the elastic valvular joint (1) faces the head (20) of the plunger (21).

9. A pre-filled syringe (2) according to claim 8, **characterized in that** the injection needle (18) is pre-installed in the exit tube (17) of the syringe (2), said exit tube (17) is closed by an internal valvular closure plug (3).

10. A pre-filled syringe (2) according to any of the above claims from 1 to 7, **characterized in that** the elastic valvular joint (1) delimits a chamber (23) that contains a solution (22) to be injected; wherein the elastic discoid plug (3) of the elastic valvular joint (1) faces the head (20) of the plunger (21).

11. A pre-filled syringe (2) according to claim10, **characterized in that** the injection needle (18) is pre-installed in the exit tube (17) of the syringe (2).

## Patentansprüche

1. Ein elastisches und gleitendes Ventilgelenk, das für den Einsatz in Fertigspritzen geeignet ist, die entweder eine innere Kammer zur Aufbewahrung des zu injizierenden Produkts oder zwei voneinander unabhängige innere Kammern mit getrennt aufbewahrten Substanzen enthalten, die jeweils vor der Injektion vermischt werden müssen. Dieses Ventilgelenk dient als temporärer Verschluss, der entweder die Verbindung zwischen der inneren Kammer und dem Verbindungskanal zur Injektionsnadel oder die Verbindung zwischen zwei inneren Kammern blockiert. Das genannte Gelenk besteht aus zwei scheibenförmigen, zusammenwirkenden Elementen, die im Inneren des Hauptkörpers der Spritze angeordnet sind und deren Umfangsrand an der zylindrischen Innenfläche abgestützt ist. Während eines der genannten Elemente ein elastischer, scheibenförmiger Stopfen ist, bildet das andere ein gleitendes, scheibenförmiges Aufnahmelager, auf dem der Stopfen über einen zylindrischen Hohlraum wirkt. Der Umfangsrand des scheibenförmigen Aufnahmelagers bildet einen Wulst, der dicker und weniger elastisch ist als der übrige Körper des genannten scheibenförmigen Aufnahmelagers. Das erwähnte Ventilgelenk ist **gekennzeichnet durch** den elastischen, scheibenförmigen Stopfen, der eine elastische Basis und einen hohlen Verschlusszylinder mit einer offenen, entfernt liegenden Basis umfasst, welcher sich aus seiner inneren Fläche heraus erstreckt. Das gleitende, scheibenförmige Aufnahmelager umfasst ein zentrales Durchgangsrohr, das dem hohlen Verschlusszylinder gegenüberliegt, sodass die elastische Basis des scheibenförmigen Stopfens lösbar in einem zylindrischen Hohlraum passt, der sich an der Öffnung des zentralen Durchgangsrohrs des gleitenden Aufnahmelagers befindet. Die elastische Basis des scheibenförmigen Stopfens weist dabei einen kleineren Durchmesser auf als das gleitende Aufnahmelager. Wenn sich der hohle Verschlusszylinder innerhalb des zentralen Durchgangsrohrs des gleitenden, scheibenförmigen Aufnahmelagers befindet, ist die elastische Basis des elastischen scheibenförmigen Stopfens in einen ringförmigen Hohlraum eingesetzt, der vom Umfangsrand des Aufnahmelagers umgeben ist, welcher den dickeren Wulst bildet. Der Außendurchmesser des hohlen Verschlusszylinders entspricht dem Innendurchmesser des zentralen Durchgangsrohrs des gleitenden, scheibenförmigen Aufnahmelagers und gewährleistet einen kalibrierten Sitz.

2. Das elastische Ventilgelenk gemäß Anspruch 1, wobei der hohle Verschlusszylinder, der sich von der elastischen Basis des elastischen, scheibenförmigen Stopfens erstreckt, alternativ einen kreisförmigen Querschnitt aufweisen kann, dessen Außendurchmesser geringfügig größer ist als der Durchmesser des zentralen Durchgangsrohrs des gleitenden, scheibenförmigen Aufnahmelagers, wodurch ein passgenauer Sitz gewährleistet wird.

3. Das elastische Ventilgelenk gemäß Anspruch 1, wobei der hohle Verschlusszylinder, der sich von der elastischen Basis des Ventilstopfens erstreckt, alternativ eine Mehrzahl externer ringförmiger Flansche aufweisen kann, die vorübergehend von der zylindrischen Innenfläche getragen werden, welche den zentralen Durchgang des gleitenden, scheibenförmigen Aufnahmelagers definiert, wodurch ein passgenauer Sitz ermöglicht wird.

4. Das elastische Ventilgelenk gemäß einem der vorstehenden Ansprüche 1 bis 3, wobei das zentrale Durchgangsrohr des gleitenden, scheibenförmigen Aufnahmelagers an seiner Öffnung mindestens zwei oberflächennahe, benachbarte Hohlräume aufweist, die einander gegenüberliegen und mit dem Inneren des zentralen Durchgangsrohrs des gleitenden, scheibenförmigen Aufnahmelagers in Verbindung stehen.

5. Das elastische Ventilgelenk gemäß einem der vorstehenden Ansprüche 1 bis 4, wobei die scheibenförmigen kooperativen Elemente in Kombination mit einem oberen Verschlussstopfen (Tip-Cap), der den Kommunikationskanal zur Injektionsnadel hin verschließt und nicht zuvor in seiner Position installiert ist, eine temporäre Verschlussvorrichtung für Fertigspritzen mit doppelten, innen koaxial angeordneten Kammern darstellen, wobei die genannten scheibenförmigen kooperativen Elemente als Trennwand zwischen diesen Kammern angeordnet sind.

6. Elastisches Ventilgelenk gemäß einem der vorstehenden Ansprüche 1 bis 4, wobei die scheibenförmigen kooperativen Elemente in Kombination mit einem Ventilverschlussstopfen, der den Kommunikationskanal zur Injektionsnadel hin innen verschließt und in seiner Position vorinstalliert ist, eine temporäre Verschlussvorrichtung für Fertigspritzen mit doppelten, innen koaxial angeordneten Kammern darstellen, wobei die genannten scheibenförmigen kooperativen Elemente als Trennwand zwischen diesen Kammern angeordnet sind.

7. Das elastische Ventilgelenk gemäß einem der vorstehenden Ansprüche 1 bis 4, wobei die scheibenförmigen kooperativen Elemente, die als temporärer Verschluss angeordnet sind, welcher verhindert, dass der Hauptkörper der Spritze intern mit dem Kommunikationskanal zur Injektionsnadel - die ggf. bereits vorinstalliert ist oder nicht - in Verbindung steht, eine temporäre Verschlussvorrichtung für Ein-Kammer-Fertigspritzen darstellen.

8. Eine Fertigspritze, die aus einem elastischen Ventilgelenk gemäß einem der vorstehendenAnsprüche 1 bis 4 besteht, welche zwei Hohlräume bildet, die jeweils ein unterschiedliches Produkt enthalten, wobei mindestens eines dieser Produkte flüssig ist. Beim Mischen der Produkte wird eine zu injizierende Lösung erhalten. Der elastische scheibenförmige Stopfen des elastischen Ventilgelenks ist dem Kopf des Kolbens zugewandt.

9. Eine Fertigspritze gemäß Anspruch 8, wobei die Injektionsnadel in der Austrittsöffnung der Spritze vorinstalliert ist. Genannte Austrittsöffnung ist durch einen inneren Ventilverschlussstopfen verschlossen.

10. Eine Fertigspritze, wobei das elastische Ventilgelenk gemäß einem der vorstehenden Ansprüche 1 bis 4 einen Hohlraum begrenzt, der eine zu injizierende Lösung enthält, und wobei der elastische, scheibenförmige Stopfen des elastischen Ventilgelenks dem Kopf des Kolbens zugewandt ist.

11. Die Fertigspritze gemäß Anspruch 9, wobei die Injektionsnadel in der Austrittsöffnung der Spritze vorinstalliert ist.

## Revendications

1. Un joint valvulaire élastique et coulissant destiné à fonctionner à l'intérieur de seringues préremplies ayant une chambre interne qui stocke le produit à injecter, ou deux chambres internes indépendantes qui contiennent des produits isolés, respectivement, et qui doivent être mélangées avant l'injection. Ce joint valvulaire agit comme une fermeture temporaire, bloquant la communication entre la chambre interne et le canal de communication vers l'aiguille d'injection, ou entre deux chambres internes. Cette articulation comprend deux éléments coopératifs discoïdes, disposés à l'intérieur du corps principal de la seringue et dont le bord périphérique est soutenu par sa surface cylindrique. L'un de ces éléments est un bouchon discoïde élastique, tandis que l'autre forme un siège de réception discoïde coulissant sur lequel le bouchon agit à travers une cavité cylindrique. Le bord périphérique du siège discoïde forme un cordon plus épais et moins élastique que le reste du corps dudit siège discoïde. Le joint valvulaire susmentionné est **caractérisé par** le bouchon discoïde élastique, qui comprend une base élastique et un cylindre de fermeture creux avec une base distante ouverte, qui est formée à partir de sa face interne. Le siège de réception discoïde coulissant comprend un tube de passage qui fait face audit cylindre de fermeture creux, de telle sorte que la base élastique du bouchon discoïde s'insère de manière amovible à l'intérieur d'une cavité cylindrique située dans l'ouverture du tube de passage central du siège de réception discoïde coulissant et où la base élastique du bouchon discoïde élastique a un diamètre inférieur à celui du siège discoïde coulissant. Lorsque le cylindre de fermeture creux se trouve à l'intérieur du tube de passage central du siège de réception discoïde coulissant, ladite base élastique du bouchon discoïde élastique est logée à l'intérieur d'une cavité circulaire entourée par le bord périphérique du siège discoïde qui forme un cordon plus épais. Le diamètre extérieur du cylindre de fermeture creux est équivalent au diamètre intérieur du tube de passage central du siège de réception discoïde coulissant, offrant ainsi un ajustement calibré.

2. Le joint valvulaire élastique selon la revendication numéro 1, dans lequel un cylindre de fermeture creux formé à partir de la base élastique du bouchon discoïde élastique peut présenter, alternativement, une section circulaire, dont le diamètre externe est légèrement supérieur au diamètre du tube de passage central du siège de réception discoïde coulissant, offrant un ajustement précis.

3. Le joint valvulaire élastique selon la revendication numéro 1, dans lequel le cylindre de fermeture creux formé à partir de la base élastique du bouchon valvulaire peut inclure, alternativement, une pluralité de brides annulaires externes qui sont temporairement maintenues par la surface cylindrique qui définit le passage central du siège de réception discoïde coulissant, offrant ainsi un ajustement précis.

4. Le joint valvulaire élastique selon les revendications précédentes de 1 à 3, dans lequel le tube de passage central du siège de réception discoïde coulissant comprend au moins deux cavités superficielles adjacentes dans son ouverture, qui sont opposées l'une à l'autre et qui communiquent avec l'intérieur du tube de passage central du siège de réception discoïde coulissant.

5. Le joint valvulaire élastique selon les revendications précédentes de 1 à 4, dans lequel les éléments discoïdaux coopératifs combinés à un bouchon supérieur (tip-cap) fermant le canal de communication vers l'aiguille d'injection, laquelle n'est pas préalablement installée dans sa position, constituent un moyen de fermeture temporaire pour des seringues préremplies à double chambres coaxiales internes, où lesdits éléments discoïdaux coopératifs sont disposés de manière à agir comme une cloison de séparation entre ces chambres.

6. Le joint valvulaire élastique selon les revendications précédentes de 1 à 4, dans lequel les éléments discoïdaux coopératifs combinés à un bouchon de fermeture valvulaire fermant intérieurement le canal de communication vers l'aiguille d'injection, laquelle est préalablement installée dans sa position, constituent un moyen de fermeture temporaire pour des seringues préremplies à double chambres coaxiales internes, où lesdits éléments discoïdaux coopératifs sont disposés de manière à agir comme une cloison de séparation entre eux.

7. Le joint valvulaire élastique selon les revendications précédentes de 1 à 4, dans lequel les éléments discoïdaux coopératifs qui sont disposés comme une fermeture temporaire empêchant le corps principal de la seringue de communiquer intérieurement avec le canal de communication vers l'aiguille d'injection, qui peut être préinstallée ou non dans sa position, sont un moyen de fermeture temporaire pour les seringues à chambre préremplie.

8. Une seringue préremplie, laquelle comprend une articulation valvulaire élastique selon les revendications 1 à 4, formant deux cavités qui contiennent chacune un produit différent et dont au moins l'un des produits est liquide. En mélangeant les produits, une solution à injecter est obtenue. Le bouchon discoïde élastique du joint valvulaire élastique fait face à la tête du piston.

9. Une seringue préremplie selon la revendication 8, dans laquelle l'aiguille d'injection est préinstallée dans le tube de sortie de la seringue. Ce tube de sortie est fermé par un bouchon de fermeture valvulaire interne.

10. Une seringue préremplie, dans laquelle le joint valvulaire élastique selon l'une des revendications précédentes de 1 à 4, délimite une cavité qui contient une solution à injecter ; et le bouchon discoïde élastique du joint valvulaire élastique fait face à la tête du piston.

11. La seringue préremplie selon la revendication 9, dans laquelle l'aiguille d'injection est préinstallée dans le tube de sortie de la seringue.
